Europäisches Patentamt

(19) European Patent Office

Office européen des brevets

(11) Publication number: **0 041 795**
**B1**

(12) **EUROPEAN PATENT SPECIFICATION**

(45) Date of publication of patent specification: **16.01.85**

(51) Int. Cl.⁴: **A 61 K 9/00**

(21) Application number: **81302327.2**

(22) Date of filing: **27.05.81**

(54) Injectable composition of rapamycin.

(30) Priority: **02.06.80 US 155250**

(43) Date of publication of application:
**16.12.81 Bulletin 81/50**

(45) Publication of the grant of the patent:
**16.01.85 Bulletin 85/03**

(84) Designated Contracting States:
**AT BE CH DE FR GB IT LI LU NL SE**

(56) References cited:
**BE-A- 877 700**
**DE-B-1 518 819**
**US-A-3 929 992**

**CHEMICAL ABSTRACTS, vol. 71, no. 11, 15th
September 1969, page 200, col. 1, no. 47895b,
Columbus Ohio (USA); T. MATSUZAWA et al.:
"Effect of some nonionic surfactants on the
absorption of enduracidin from the muscles"**

(73) Proprietor: **AYERST, MCKENNA AND
HARRISON INC.**
**1025 Laurentian Boulevard
St. Laurent Quebec H4R 1J6 (CA)**

(72) Inventor: **Sehgal, Surendra Nath**
**206 Sherwood Road
Beaconsfield Quebec H9W 2G8 (CA)**
Inventor: **Baker, Harold Arthur**
**11806 DeMeulles
Montreal Quebec, H4J 2E5 (CA)**
Inventor: **Sidorowicz, Anne Marie**
**4350 Mariette Avenue
Montreal Quebec, H4D 2E9 (CA)**
Inventor: **Vezina, Claude**
**11, Rue St. Sulpice
Oka Quebec, J0N 1E0 (CA)**

(74) Representative: **Wileman, David Francis et al
c/o John Wyeth and Brother Limited
Huntercombe Lane South
Taplow Maidenhead Berkshire SL6 OPH (GB)**

The file contains technical information
submitted after the application was filed and
not included in this specification

Courier Press, Leamington Spa, England.

(56) References cited:
**CHEMICAL ABSTRACTS, vol. 77, no. 6, 7th August 1972, page 325, col. 2 - page 326, col. 1, no. 39130g, Columbus,Ohio (USA); A.A. KASSEM et al.: "Solubilization of griseofulvin as a function of low temperature and different nonionic surface-active agents"**
**"Index of antibiotics from actinomycetes", vol. II, H.Humezawa, Japan scientific societies press, 1978, page 869, Tokyo (JP).**

**Description**

This invention relates to a novel injectable composition of rapamycin which provides a high order of rapamycin in the blood of a mammal following injected administration of the composition. More specifically, the herein disclosed invention relates to new compositions of rapamycin containing a nonionic surfactant. The invention further relates to a process for preparing and a method for using the disclosed composition.

Rapamycin is an antifungal antibiotic described by C. Vezina *et al.*, J. Antibiot., *28*, 721 (1975), S. N. Sehgal *et al.*, J. Antibiot., *28*, 727 (1975), S. N. Sehgal *et al.*, U.S. Patents 3,929,992, issued December 30, 1975 and S. N. Sehgal *et al.*, U.S. Patent 3,993,749, issued November 23, 1976. Rapamycin is extracted from a streptomycete (*Streptomyces hygroscopicus* NRRL 5491) isolated from an Easter Island soil sample and is particularly effective against *Candida albicans* both in vitro and in vivo, H. A. Baker *et al.*, J. Antibiot., *31*, 539 (1978). *Streptomyces hygroscopicus* NRRL 5491 samples were deposited 23rd June 1972 without restrictions with the Northern Utilization and Research Division, Agricultural Research Service, U.S. Department of Agriculture, Peoria, Illinois, U.S.A. A report by R. R. Martel *et al.*, Can. J. Physiol., *55*, 48 (1977) describes the use of rapamycin for the prevention of the development of experimental immunopathies. Recently rapamycin was shown to be an effective agent for treating carcinogenic tumors in a mammal by S. N. Sehgal and C. Vezina, Belgian Patent No. 877700, issued January 14, 1980.

Rapamycin and compositions thereof, as described in the above reports, are virtually insoluble in aqueous solutions and are not suitable for injection. The composition of this invention provides rapamycin in a water soluble formulation which is suitable for intravenous injection.

A number of drugs having poor water solubility have been formulated with nonionic surfactants. Polyoxyethylated glycerin hydroxy fatty acids, such as sold under the trade mark Cremophor, are examples of such nonionic surfactants. Cremophor was shown to be an effective solvent for the prevention of i.v. diazepin-induced thrombophlebitis by M.A.K. Mattila *et al.*, Br. J. Anaesth., *51*, 891 (1979). Cremophor was used as a solubilizing agent for an injectable anaesthetic by B. Kay and G. Rolly, Acta. Anaesth. Belg., *28*, 303 (1977) and B. Kay and G. Rolly, Acta. Anaesth, Belg., *28*, 317 (1977). The antifungal agent, Monistat i.v., is formulated with PEG 40 castor oil, "Physicians' Desk Reference", Publisher Charles E. Baker, Jr., Published by Medical Economics Company, Oradel, New Jersey, 33 ed., 1979, p. 1255. Formulations of ergot alkaloids with a large number of nonionic emulsifiers are described by J. Franz, United Kingdom Patent 1,513,383, published June 7, 1978. Suggested uses and technical description of the Cremophor surfactants are described in the following Technical Leaflets: "Cremophor EL", M 1714 e, (7063) May 1977 (RB), 5th Revision, BASF Aktiengesellscaft, D-6700 Ludwigshafen, Federal Republic of Germany and "Cremophor RH Grades", M 5629 e, April 1978 (JWF), BASF Aktiengesellschaft, D-6700 Ludwigshafen, Federal Republic of Germany. Rapamycin, per se, does not readily go into solution in polyoxyethylated glycerin hydroxy fatty acids. The present invention relates to a new injectable composition of rapamycin which is neither disclosed in, nor rendered obvious by, any of the above cited reports, or other references of which we are aware.

According to the present invention, an injectable composition of rapamycin suitable for intravenous administration, is provided comprising 1 to 20 milligrams of rapamycin per millilitre of the composition and a nonionic surfactant selected from polyoxyethylated fatty acids, polyoxyethylated fatty alcohols or polyoxyethylated glycerine hydroxy fatty acid esters. A preferred injectable composition comprises a therapeutically effective amount of rapamycin in an aqueous solution of 1 to 50 per cent by weight of the nonionic surfactant. A more preferred injectable composition contains an aqueous solution of 1 to 35 per cent by weight of the nonionic surfactant. A most preferred composition contains an aqueous solution of 1 to 20 per cent by weight of the nonionic surfactant. The above compositions can also contain pharmaceutically acceptable excipients commonly used in pharmaceutical formulations.

The injectable rapamycin composition of this invention provides a therapeutically effective amount of rapamycin in the blood of a mammal and also provides a therapeutically effective amount of rapamycin in the brain, liver, kidney, lung and spleen of a mammal.

The injectable rapamycin composition comprises the following three main components: rapamycin, a nonionic surfactant and water.

The therapeutically effective amount of rapamycin in the injectable composition is usually in the range of 1 to 20 mg/ml of solution.

The nonionic surfactant can be in the range of 1 to 50 per cent by weight of the injectable composition. In good pharmaceutical practice, the amount of solubilizing agents and excipients should be kept at a minimum, thus the amount of the nonionic surfactant in the composition should be dependent on the desired amount of rapamycin in the composition.

Nonionic surfactants used in the injectable composition of rapamycin increase the solubility of rapamycin in aqueous solutions. Two or more nonionic surfactants can also be used in the injectable composition of rapamycin. Nonionic surfactants for use in the composition are selected from polyoxyethylated fatty acids; polyoxyethylated fatty alcohols; and polyoxyethylated glycerin hydroxy fatty acid esters, e.g. polyoxyethylated castor oil, exemplified by Cremophor EL and polyoxyethylated hydrogenated castor oil, exemplified by Cremophor RH and Cremophor RH 60. Polyoxyethylated castor oil is prepared by reacting ethylene oxide with castor oil, for example, Cremophor EL is the product obtained from the reaction of 35 moles of the ethylene oxide with one mole of castor oil. Similarly, polyoxyethylated hydrogenated castor oil is obtained by reacting ethylene oxide with hydrogenated castor oil, for example Cremophor RH 60 is prepared by reacting 60 moles of ethylene oxide with one mole of hydrogenated castor oil. In a similar manner, other polyoxyethylated fatty acids or fatty alcohols can be prepared by the reaction of ethylene oxide with a fatty acid, for example, stearic acid or with a fatty alcohol, for example, cetyl alcohol. Preferred nonionic surfactants in the composition are selected from polyoxyethylated castor oil and polyoxyethylated hydrogenated castor oil.

The rapamycin composition of the invention is prepared by the discovery of a method to dissolve rapamycin in a nonionic surfactant so that when diluted with water, rapamycin remains in solution. Rapamycin is insoluble in most surfactants, including the nonionic surfactants. Rapamycin is first dissolved in an organic solvent miscible with the surfactant; preferably the solvent is one that can be removed easily by distillation. Examples of these solvents are acetone, methanol, and ethanol. Thereafter the rapamycin containing solvent and the surfactant are admixed. If desired or if the solvent is not pharmaceutically acceptable, the solvent is removed from the solution containing rapamycin, nonionic surfactant and solvent. A preferred method of removing the solvent is by evaporation wherein the solution is subjected to reduced pressure at 20 to 50°C until the solution remains at constant weight. Upon removal of the solvent, the rapamycin remains in solution in the nonionic surfactant. Dilution of the above solutions containing rapamycin, solvent and nonionic surfactant or rapamycin and nonionic surfactant with water or an aqueous solution containing pharmaceutically acceptable excipients provides compositions suitable for injection. This dilution is preferably made shortly before administration, e.g. within four hours before injection. Another mode of preparing the injectable solution would be to admix a solution of the surfactant in water with the rapamycin-containing organic solvent.

Accordingly this invention provides a process for preparing an injectable rapamycin composition as hereinbefore defined which comprises dissolving rapamycin in an organic solvent which is miscible with the nonionic surfactant and either

(i) adding the nonionic surfactant, if required removing some or all of the organic solvent, and adding water; or

(ii) adding a solution of nonionic surfactant in water;

the amounts of rapamycin and nonionic surfactant being predetermined to give the desired concentrations in the composition.

Pharmaceutically acceptable excipients as used herein are the additives which provide isotonic composition, for example, saline, Ringer's solution and glucose; and preservatives.

The rapamycin composition can also contain other nonionic surfactants, for example, polysorbate 80, propylene glycol and/or polyethylene glycol.

The following examples illustrate further this invention where percentages relating to final compositions are on a w/w basis.

Example 1
Preparation of injectable rapamycin compositions

A. Rapamycin compositions without organic solvent
Rapamycin (100 mg) was dissolved in ethanol (4 ml) and to this solution, polyoxyethylated castor oil (Cremophor EL, 2 ml≡2g, density=1.05 g/ml @ 25°C) was added. The solution was subjected to evaporation under reduced pressure at 50°C until the ethanol was removed to give a solution containing 50 mg of rapamycin per millilitre of Cremophor EL. To 2 ml of the latter solution, water (8 ml) was added to provide a solution containing 10 mg of rapamycin per millilitre of aqueous 20% Cremophor EL.

In the same manner, but replacing ethanol with an equivalent amount of acetone or methanol, the latter solution of rapamycin was obtained.

Similarly, by replacing water with physiological saline, a solution containing 10 mg of rapamycin and 20% Cremophor EL in physiological saline is obtained.

Similarly, by using 10, 20, 50, 150 or 200 mg of rapamycin, the following solutions are obtained, respectively: 1 mg of rapamycin per millilitre of aqueous 20% Cremophor EL, 2 mg of rapamycin per millilitre of aqueous 20% Cremophor EL, 5 mg of rapamycin per millilitre of aqueous 20% Cremophor EL, 15 mg of rapamycin per millilitre of aqueous 20% Cremophor EL, and 20 mg of rapamycin per millilitre of aqueous 20% Cremophor EL.

Similarly, by replacing polyoxyethylated castor oil with an equivalent volume of polyoxyethylated hydrogenated castor oil (Cremophor RH 40 and Cremophor RH 60) or a mixture containing equal volumes of polyoxyethylated castor oil (Cremophor EL) and polysorbate 80, the following injectable compositions are obtained, respectively: 10 mg of rapamycin per millilitre of aqueous 20% Cremophor

4

RH 40, 10 mg of rapamycin per millilitre of aqueous 20% Cremophor RH 60, and 10 mg of rapamycin per millilitre of aqueous 10% Cremophor EL and 10% polysorbate 80.

Similarly, by using 20 mg of rapamycin and 1 ml of Cremophor EL or 50 mg of rapamycin and 2 ml of Cremophor EL, the following injectable compositions are obtained, respectively: 1 mg of rapamycin per millilitre of aqueous 5% Cremophor EL and 5 mg of rapamycin per millilitre of aqueous 10% Cremophor EL.

B. Rapamycin compositions with organic solvent

Rapamycin (1.0 g) was dissolved in a solution of ethanol (8.0 g), propylene glycol (32.0 g) and Cremophor RH (10.0 g). To this solution, was added 50.0 g of water. The resulting solution (100 ml) provided a composition containing 10 mg of rapamycin per millilitre of a solution composed of 8% ethanol, 32% propylene glycol, 10% Cremophor RH and 50% water.

Similarly, by using 1.5 g of rapamycin, the following injectable composition was obtained, 15 mg of rapamycin per millilitre of a solution composed of 8% ethanol, 32% propylene glycol, 10% Cremophor RH and 50% water.

C. Concentrated rapamycin solution with a surfactant diluent

Rapamycin (67.98 g) and butylated hydroxyanisole (1.24 g), as an antioxidant preservative, were dissolved in 930.78 g of nitrogen purged absolute ethanol. The solution was filtered through a bacterial filter and 1.1 ml of filtrate was filled into a 5 ml-amber glass ampule using a nitrogen flush. The ampule was sealed to protect the concentrated rapamycin solution from light and air. Benzyl alcohol N. F. (33.34 g), as an antiseptic, and Cremophor EL (111.12 g) were added to sufficient water (USP) with mixing to make 1000 ml of a surfactant diluent solution. The diluent solution was filtered and 9 ml of the filtrate was filled into a 20 ml glass vial. Within four hours of injection, 1.0 ml of the contents of the ampule of concentrated rapamycin solution is withdrawn, via a syringe, added to a vial of diluent solution, and shaken to form an injectable solution containing 5.5 mg rapamycin per ml.

Example 2
Assay method for determination of rapamycin in the serum and tissue of an animal

A. Serum concentrations

Medium:

Nystatin assay agar (Antibiotic Medium 12), BBL 10,982. The test organism, *Candida Albicans* House AY F-598, was maintained by weekly transfer on Sabouraud's agar slopes. Slope cultures were incubated for 48 hours at 30°C and stored at 4°C until used. Test inoculum was prepared by subculturing the *Candida* strain in Sabouraud's broth and incubating overnight at 37°C.

Preparation of plates:

In the assay, a modification of the antibiotic assay method described by J. V. Bennett *et al.*, Applied Microbiology, *14* (2), 170 (1966) was used. 200 Millilitre aliquots of molten Nystatin assay agar kept at 56°C were inoculated with 0.25 ml of a $10^{-1}$ dilution of an overnight broth culture of *C. albicans* F-598. When the seeded agar solidified, 64 wells of 4.7 mm in diameter were punched at spaced intervals on an agar surface measuring 12"×12"; this size of wells gave suitable zone diameters with the range of standards used. After the wells were filled with standards and samples, plates were covered, sealed and incubated for 18 hours at 37°C.

Standards:

Rapamycin powder was weighed out in convenient amounts, and a stock solution prepared in methanol to give a concentration of 1,000 μg/ml; further dilutions were carried out in appropriate serum. Final concentration of standards in serum were: 5.0, 2.5, 1.25, 0.62 and 0.31 μg/ml respectively. Averages of a minimum of four separate zone diameter readings per standard concentration were plotted on semilog 3-cycle graph paper against the antibiotic concentrations. These five points served to draw the best fitting straight line. The concentrations of the unknown samples were calculated by obtaining the average diameters of at least four inhibition zones per sample and converting them to concentration by interpolation from the standard curve.

B. Sample preparation:

Similar to procedure for serum concentration.

Standards:

A stock solution of rapamycin containing 1000 μg/ml in methanol was prepared. Further dilutions were carried out in methanol to give concentrations of 500, 250, 125, 62.5, 31.0 and 15.5 μg/ml, respectively. 0.1 Millilitre of the above concentrations was added to 1 gram sample of the various tissues: lung, liver, kidney, spleen and brain. To this mixture, 3.9 ml of saline was added to give a final concentration of 10, 5, 2.5, 1.25, 0.62 and 0.31 $\mu$g/gram of tissue. All samples were mixed by means of a Virtis No. 45 homogenizer, Virtis Research Equipment, Gardiner, New York. Averages of a minimum of four separate zone diameter readings per standard concentration were plotted on semi-log 3-cycle graph paper against the rapamycin concentrations. These five points served to draw the best fitting straight line. The concentrations of the unknown samples were calculated by obtaining the average diameters of at least four inhibition zones per sample and converting them to concentrations by interpolation from the standard curve.

C. Sample collection

All formulations were prepared in the solvent and diluted with sterile distilled water prior to administration. Mice were sacrificed and bled by cardiac puncture and the following organs removed for tissue studies: brain, lung, liver, kidney and spleen. Standard curves for blood and tissue concentrations were prepared in the appropriate serum and tissue. Control mice (10) were administered each rapamycin formulations and were observed for a period of 60 days for deaths.

Sample preparation:

A one-gram sample was placed in a 30-ml Virtis Homogenizer cup and to this was added 4 ml of a 2% methanol in saline solution and the tissue was ground by the Virtis No. 45 Homogenizer to aid in sampling and to break down some of the connective tissue. This solution was used in the microbiological assay described above.


Example 3
Administration of the rapamycin injectable compositions and results

A. Test animals and mode of administration
    Mice, Swiss strain DC-1 (CBL, Montreal), 30 grams, male.
        Intraperitoneal, 0.5 ml, peritoneal cavity.
        Intravenous, 0.2 ml, tail vein.
    Dog, beagle, 7.0 kg, female.
        Intravenous administration, 5 ml, slow infusion, tibial vein.
    Rat, Sprague Dawley (CBL, Montreal), 80 grams, male.
        Intravenous administration, 0.5 ml, caudal vein.


B. Rapamycin in 8% ethanol plus 32% propylene glycol plus 10% cremophor RH 40 plus 50% water
    Concentration of rapamycin: 10 mg and 15 mg/ml.
    Test animal: Mice, 6 per sample time for serum and tissue concentrations.
        Dog, one sample per time interval.

    Doses and administration: Mice 66.5 and 99.7 mg/kg single intravenous injection.
        Dog, 7.1 mg/kg, single, slow intravenous injection.
    Sample times: Mice 15, 30, 60 minutes, 1, 2, 4, 6, 7 and 8 hours.
        Dog, 15 30, 60 minutes, 2 and 4 hours
    Assays: Mice, serum and tissue concentrations at sample times listed above.
        Dog, serium concentrations at sample times listed above.
    Results: Results of serum concentrations for doses of 66.5 and 99.7 mg/kg in mice are reported in Tables 1 and 2. Tissue concentrations in mice are reported in Tables 1 and 2. Serum concentrations in a dog are reported in Table 3.


C. Rapamycin in 20% cremophor EL plus 80% water
    Concentration of rapamycin: 10 mg/ml.
    Test animal: Mice, 6 per sample time for serum and tissue concentrations.
    Doses and administration: Mice 33.3 and 66.5 mg/kg single intravenous injection.
    Sample times: 15, 30 and 60 minutes, 2, 4, 6 and 24 hours.
    Assays: Serum and tissue concentrations at sample times listed above.
    Results: The result of the serum concentrations and tissue concentrations obtained with the doses of 33.3 and 66.5 mg/kg in mice are reported in Tables 4 and 5 respectively.


D. Rapamycin in 8% ethanol plus 32% propylene glycol plus 10% cremophor RH 40 plus 50% water
    Concentration of rapamycin: 10 mg/ml.
    Test animal: Mice, 6 per sample time for serum and tissue concentrations.
    Dose and administration: 160 mg/kg, single intraperitoneal injection.
    Sample times: 30, 60 minutes, 2, 4, 6 and 24 hours.
    Assays: Serum and tissue concentrations at sample times listed above.
    Results: Results of the serum concentration and tissue concentrations are reported in Table 6.

6

E. Rapamycin in 20% cremophor EL plus 80% water
Concentration of rapamycin: 10 mg/ml.
Test animal: Rat, 2 per sample time.
Doses and administration: Rat, 62.5 mg/kg, single intravenous injection.
Sample times: 15, 30 and 60 minutes, 2, 4 and 6 hours.
Assays: Tissue concentrations at sample times listed above.
Results: Results of the tissue concentrations are reported in Table 7.

F. Rapamycin in 20% cremophor EL plus 80% water
Concentration of rapamycin: 7.5 mg/ml.
Test animal: Rat, 2 per sample time.
Doses and administration: Rat, 46.8 mg/kg, single intravenous injection.
Sample times: 15, 30 and 60 minutes, 2, 4 and 6 hours.
Assays: Serum and tissue concentrations at sample times listed above.
Results: Results of the serum concentrations and tissue concentrations are reported in Table 8.

G. Rapamycin in 20% Cremophor EL plus 80% water
Concentration of rapamycin: 5.0 mg/ml.
Test animal: Rat, 2 per sample time.
Doses and administration: Rat, 31.25 mg/kg, single intravenous injection.
Sample times: 15, 30 and 60 minutes, 2, 4 and 6 hours.
Assays: Serum and tissue concentrations at sample times listed above.
Results: Results of the serum concentrations and tissue concentrations are reported in Table 9.

TABLE 1

Serum and tissue concentration after intravenous administration of rapamycin in 8% ethanol, 32% propylene glycol, 10% cremophor RH 40 and 50% water in mice at a dose of 66.5 mg/kg.

| Time | Serum[a] | Brain[b] | Liver[b] | Kidney[b] | Lung[b] | Spleen[b] |
|---|---|---|---|---|---|---|
| 15 min | 19.5 | 1.32 | 8.8 | 17.0 | 5.7 | 2.0 |
| 30 ,, | 13.5 | 1.05 | 7.0 | 8.4 | 4.3 | 1.6 |
| 60 ,, | 7.9 | 0.49 | 5.1 | 5.6 | 2.7 | 0.86 |
| 2 hr | 3.6 | 0.41 | 1.8 | 2.0 | 1.5 | 0.21 |
| 4 ,, | 2.35 | 0 | 0.46 | 0.97 | 0.76 | 0.1 |
| 6 ,, | 2.0 | 0 | 0.43 | 0.55 | 0.66 | 0.1 |
| 7 ,, | 2.77 | 0 | trace | 0.32 | 0.72 | trace |
| 8 ,, | 1.3 | 0 | 0 | 0 | 0.4 | 0 |
| 24 ,, | — | — | — | — | — | — |

[a] Rapamycin in Serum, $\mu$g/ml
[b] Rapamycin in Tissues, $\mu$g/g

TABLE 2

Serum and tissue concentrations after intravenous administration of rapamycin in 8% ethanol, 32% propylene glycol, 10% cremophor RH 40 and 50% water in mice at a dose of 99.7 mg/kg

| Time | Serum[a] | Brain[b] | Liver[b] | Kidney[b] | Lung[b] | Spleen[b] |
|------|----------|----------|----------|-----------|---------|-----------|
| 15 min | 29.2 | 1.7 | 14.2 | 11.2 | 7.0 | 10.0 |
| 30 ,, | 26.5 | 1.7 | 8.0 | 11.0 | 5.2 | 6.7 |
| 60 ,, | 12.5 | 1.25 | 6.4 | 7.5 | 5.6 | 2.5 |
| 2 hr | 6.0 | 1.02 | 4.5 | 4.5 | 4.6 | 2.25 |
| 4 ,, | 3.4 | 0.49 | 2.85 | 2.95 | 5.0 | 0.8 |
| 6 ,, | 2.5 | trace | 0.62 | 0.53 | 7.4 | 0.1 |
| 7 ,, | — | — | — | — | — | — |
| 8 ,, | — | — | — | — | — | — |
| 24 ,, | — | — | — | — | — | — |

[a] Rapamycin in Serum, $\mu$g/ml
[b] Rapamycin in Tissues, $\mu$g/g

TABLE 3

Serum concentration after intravenous administration of rapamycin in 8% ethanol, 32% propylene glycol, 10% cremophor RH 40 and 50% water in a dog at a dose of 7.1 mg/kg

| Time | Serum[a] |
|------|----------|
| 0 | 0 |
| 15 min | 2.3 |
| 30 min | 2.3 |
| 1 hr | 0.95 |
| 2 hr | 0.34 |
| 4 hr | 0.135 |

[a] Rapamycin in Serum, $\mu$g/ml

### TABLE 4
Serum and tissue concentrations after intravenous administration of rapamycin in
20% cremophor EL and 80% water to mice at a dose of 33.3 mg/kg

| Time | Serum[a] | Brain[b] | Liver[b] | Kidney[b] | Lung[b] | Spleen[b] |
|---|---|---|---|---|---|---|
| 15 min | 6.4 | 0 | 9.0 | 9.2 | 7.5 | 2.1 |
| 30 ,, | 6.45 | 0 | 11.0 | 3.75 | 8.0 | 1.35 |
| 60 ,, | 3.45 | 0 | 5.2 | 1.9 | 4.0 | 0.62 |
| 2 hr | 3.47 | 0 | 2.0 | 0.44 | 1.2 | 0.26 |
| 4 ,, | 1.65 | 0 | 0.86 | 0.16 | 0.56 | 0.1 |
| 6 ,, | 2.2 | 0 | 0.4 | Trace | 0.28 | 0 |
| 24 ,, | 0 | 0 | 0 | 0 | 0 | 0 |

[a] Rapamycin in Serum, $\mu$g/ml
[b] Rapamycin in Tissues, $\mu$/g

### TABLE 5
Serum and tissue concentrations after intravenous administration of rapamycin
in 20% cremophor EL and 80% water to mice at a dose of 66.5 mg/kg.

| Time | Serum[a] | Brain[b] | Liver[b] | Kidney[b] | Lung[b] | Spleen[b] |
|---|---|---|---|---|---|---|
| 15 min | 18.0 | 0.68 | 16.0 | 10.0 | 19.0 | 8.3 |
| 30 ,, | 12.35 | 0.83 | 8.5 | 6.1 | 14.5 | 7.5 |
| 60 ,, | 11.75 | 0.4 | 12.5 | 7.0 | 10.0 | 3.75 |
| 2 hr | 6.6 | 0.5 | 5.2 | 2.85 | 12.0 | 2.5 |
| 4 ,, | 5.0 | 0 | 4.2 | 1.15 | 1.45 | 0.56 |
| 6 ,, | 2.77 | 0 | 0.27 | 0.38 | 0.58 | 0.155 |
| 24 ,, | 0 | 0 | 0 | 0 | 0 | 0 |

[a] Rapamycin in Serum, $\mu$g/ml
[b] Rapamycin in Tissues, $\mu$g/g

### TABLE 6
Serum and tissue concentrations after intraperitoneal administration of rapamycin
in 8% ethanol, 32% propylene glycol, 10% cremophor RH 40 and water
in mice at a dose of 160 mg/kg.

| Time | Serum[a] | Brain[b] | Liver[b] | Kidney[b] | Lung[b] | Spleen[b] |
|------|----------|----------|----------|-----------|---------|-----------|
| 15 min | — | — | — | — | — | — |
| 30 „ | 8.85 | 0 | 6.5 | 7.2 | 8.0 | 6.4 |
| 60 „ | 17.0 | 0 | 10.0 | 4.3 | 15.3 | 6.4 |
| 2 hr | 16.75 | 0 | 10.0 | 11.0 | 11.2 | 5.1 |
| 4 „ | 10.65 | 0 | 6.2 | 12.2 | 7.1 | 5.1 |
| 6 „ | 7.15 | — | 1.8 | 6.7 | 3.4 | 5.5 |
| 7 „ | — | — | — | — | — | — |
| 8 „ | — | — | — | — | — | — |
| 24 „ | 0.68 | 0 | 0 | 0 | 0.4 | 0.62 |

[a] Rapamycin in Serum, $\mu$g/ml
[b] Rapamycin in Tissues, $\mu$g/g

### TABLE 7
Tissue concentrations after intravenous administration of rapamycin in 20%
cremophor EL and 80% water to rats at a dose of 62.5 mg/kg

| Time | Rapamycin ($\mu$g/g) | | | | |
|------|-------|-------|---------|-------|--------|
| | Brain | Liver | Kidneys | Lungs | Spleen |
| 0 | 0 | 0 | 0 | 0 | 0 |
| 15 min | 1.95 | 48.0 | 19.0 | 16.5 | 15.0 |
| 30 „ | 0.76 | 40.5 | 19.0 | 16.0 | 12.0 |
| 60 „ | not available | 16.0 | 6.7 | 9.2 | 4.05 |
| 2 hrs | not available | 13.2 | 7.2 | 8.4 | 4.45 |
| 4 „ | 0.76 | 8.8 | 3.8 | 4.05 | 3.2 |
| 6 „ | <0.6 | 6.0 | 3.2 | 3.0 | 1.8 |

**0041795**

TABLE 8

Serum and tissue concentrations after intravenous administration of rapamycin in 20% cremophor EL and 80% water to rats at a dose of 46.8 mg/kg

| Time | Serum[a] | Brain[b] | Liver[b] | Kidney[b] | Lung[b] | Spleen[b] |
|------|----------|----------|----------|-----------|---------|-----------|
| 15 min | 10.0[c]—10.0[c] | 0 | 43.0 | 13.8 | 13.0 | 8.3 |
| 30 „ | 10.5—10.5 | 0 | 31.0 | 12.0 | 14.0 | 8.3 |
| 60 „ | 7.6—3.1 | 0.83 | 12.0 | 4.95 | 7.2 | 4.4 |
| 2 hr | 6.0—2.7 | 0 | 5.2 | 2.15 | 3.0 | 1.6 |
| 4 „ | 2.2—1.25 | 0 | 5.2 | 2.15 | 3.0 | 1.6 |
| 6 „ | 0.45—1.0 | 0 | 5.2 | 2.15 | 3.0 | 1.6 |

[a] Rapamycin in Serum, $\mu g/ml$
[b] Rapamycin in Tissues, $\mu g/g$
[c] Values from individual rats

TABLE 9

Serum and tissue concentrations after intravenous administration of rapamycin in 20% cremophor EL and 80% water to rats at a dose of 31.25 mg/kg

| Time | Serum[a] | Brain[b] | Liver[b] | Kidney[b] | Lung[b] | Spleen[b] |
|------|----------|----------|----------|-----------|---------|-----------|
| 15 min | 2.4[c]—6.3[c] | 0 | 5.2 | 8.6 | 5.2 | 3.3 |
| 30 „ | 6.4—3.5 | 0 | 12.5 | 6.7 | 6.4 | 3.8 |
| 60 „ | N.A.—3.6 | 0 | 8.7 | 5.0 | 6.3 | 2.3 |
| 2 hr | 2.0—3.35 | 0 | 5.6 | 3.5 | 4.4 | 2.0 |
| 4 „ | 0.84—0.19 | 0 | 1.4 | 1.4 | 1.03 | 0.72 |
| 6 „ | 0.17—0.19 | 0 | 1.1 | 0.76 | 0.7 | 0.475 |

[a] Rapamycin in Serum, $\mu g/ml$
[b] Rapamycin in Tissues, $\mu g/ml$
[c] Values from individual rats
N.A.=not available

**Claims for the Contracting States BE CH DE FR GB IT LI LU NL SE**

1. An injectable composition of rapamycin, suitable for intravenous administration, which comprises 1 to 20 milligrams of rapamycin per millilitre of the composition and a nonionic surfactant selected from polyoxyethylated fatty acids, polyoxyethylated fatty alcohols and polyoxyethylated glycerine hydroxy fatty acid esters.

2. A composition of Claim 1 containing an aqueous solution of 1 to 50 percent by weight of the nonionic surfactant.

3. A composition of Claim 1 containing an aqueous solution of 1 to 20 percent by weight of the nonionic surfactant.

4. A composition as claimed in Claim 1 or Claim 2 wherein the nonionic surfactant is polyoxyethylated castor oil or polyoxyethylated hydrogenated castor oil.

5. A composition of Claim 1 containing 1 to 20 milligrams of rapamycin per millilitre of an aqueous solution of 1 to 20 percent by weight of a nonionic surfactant selected from polyoxyethylated castor oil and polyoxyethylated hydrogenated castor oil.

6. A process for preparing an injectable composition as claimed in Claim 1 which comprises dissolving rapamycin in an organic solvent which is miscible with the nonionic surfactant, and either

11

(i) adding the nonionic surfactant, if required removing some or all of the organic solvent, and adding water; or

(ii) adding a solution of nonionic surfactant in water;

the amounts of rapamycin and nonionic surfactant being predetermined to give the desired concentrations in the composition.

7. A process as claimed in Claim 6 wherein the organic solvent is acetone, methanol or ethanol.

8. A process as claimed in Claim 6 or Claim 7 wherein the nonionic surfactant is polyoxyethylated castor oil or polyoxyethylated hydrogenated castor oil.

9. A process as claimed in any one of Claims 6, 7 and 8 wherein the aqueous solution comprises 1 to 20 percent by weight of nonionic surfactant.

10. An intravenously injectable composition as claimed in any one of Claims 1 to 5 for use as an agent for enhancing blood levels of rapamycin or for providing rapamycin in the brain of a mammal.

**Claims for the Contracting State AT**

1. A process for preparing an injectable composition, suitable for intravenous administration, comprising 1 to 20 milligrams of rapamycin per millilitre of an aqueous solution of a nonionic surfactant selected from polyoxyethylated fatty acids, polyoxyethylated fatty alcohols and polyoxyethylated glycerin hydroxy fatty acid esters, which comprises dissolving rapamycin in an organic solvent which is miscible with the nonionic surfactant, and either

(i) adding the nonionic surfactant, if required removing some or all of the organic solvent, and adding water; or

(ii) adding a solution of nonionic surfactant in water;

the amounts of rapamycin and nonionic surfactant being predetermined to give the desired concentrations in the composition.

2. A process as claimed in Claim 1 wherein the organic solvent is acetone, methanol or ethanol.

3. A process as claimed in Claim 1 or Claim 2 wherein the nonionic surfactant is polyoxyethylated castor oil or polyoxyethylated hydrogenated castor oil.

4. A process as claimed in Claim 1 or Claim 2 wherein the aqueous solution comprises 1 to 20 percent by weight of nonionic surfactant.

5. A process as claimed in Claim 1 in which the aqueous solution comprises 1 to 20 percent by weight of a nonionic surfactant selected from polyoxyethylated castor oil and polyoxyethylated hydrogenated castor oil.

**Patentansprüche für die Vertragsstaaten: BE, CH, DE, FR, GB, IT, LI, LU, NL, SE:**

1. Für intravenöse Verabreichung geeignete injizierbare Zusammensetzung, welche 1 bis 20 mg Rapamycin pro ml Zusammensetzung und ein nichtionisches Tensid ausgewählt aus polyoxyäthylierten Fettsäuren, polyoxyäthylierten Fettalkoholen und polyoxyäthylierten Glycerinhydroxyfettsäureestern aufweist.

2. Zusammensetzung gemäß Anspruch 1, die eine wässerige Lösung von 1 bis 50%-Masse des nichtionischen Tensids enthält.

3. Zusammensetzung gemäß Anspruch 1, die eine wässerige Lösung von 1 bis 20%-Masse des nichtionischen Tensids enthält.

4. Zusammensetzung, wie in Anspruch 1 oder Anspruch 2 beansprucht, worin das nichtionische Tensid polyoxyäthyliertes Rizinusöl oder polyoxyäthyliertes hydriertes Rizinusöl ist.

5. Zusammensetzung gemäß Anspruch 1, die 1 bis 20 mg Rapamycin pro ml wässeriger Lösung von 1 bis 20%-Masse eines nichtionischen Tensids ausgewählt aus polyoxyäthyliertem Rizinusöl und polyoxyäthyliertem hydrierten Rizinusöl enthält.

6. Verfahren zum Herstellen einer injizierbaren Zusammensetzung, wie in Anspruch 1 beansprucht, welches des Lösen von Rapamycin in einem organischen Lösungsmittel, das mit dem nichtionischen Tensid mischbar ist, und entweder

(i) Zusetzen des nichtionischen Tensids, wenn erforderlich, Entfernen eines Teils oder des gesamten organischen Lösungsmittels und Zugeben von Wasser, oder

(ii) Zusetzen einer Lösung von nichtionischem Tensid in Wasser umfaßt, wobei die Anteile an Rapamycin und nichtionischem Tensid vorherbestimmt werden, um in der Zusammensetzung die gewünschten Konzentrationen zu ergeben.

7. Verfahren, wie in Anspruch 6 beansprucht, worin das organische Lösungsmittel Aceton, Methanol oder Äthanol ist.

8. Verfahren, wie in Anspruch 6 oder Anspruch 7 beansprucht, worin das nichtionische Tensid polyoxyäthyliertes Rizinusöl oder polyoxyäthyliertes hydriertes Rizinusöl ist.

9. Verfahren, wie in einem der Ansprüche 6, 7 und 8 beansprucht, worin die wässerige Lösung 1 bis 20%-Masse nichtionisches Tensid aufweist.

10. Intravenös injizierbare Zusammensetzung, wie in einem der Ansprüche 1 bis 5 beansprucht, zur Verwendung als Mittel zum Erhöhen der Blutspiegel an Rapamycin oder zum Vorsehen von Rapamycin in Gehirn eines Säugers.

**Patentansprüche für der Vertragsstaat AT:**

1. Verfahren zum Herstellen einer für intravenöse Verabreichung geeigneten injizierbaren Zusammensetzung, die 1 bis 20 mg Rapamycin pro ml einer wässerigen Lösung eines nichtionischen Tensids ausgewählt aus polyoxyäthylierten Fettsäuren, polyoxyäthylierten Fettalkoholen und polyoxyäthylierten Glycerinhydroxyfettsäureestern aufweist, welches das Lösen von Rapamycin in einem organischen Lösungsmittel, das mit dem nichtionischen Tensid mischbar ist, und entweder

(i) Zusetzen des nichtionischen Tensids, wenn erforderlich, Entfernen eines Teils oder des gesamten organischen Lösungsmittels und Zugeben von Wasser, oder

(ii) Zusetzen einer Lösung von nichtionischem Tensid in Wasser umfaßt, wobei die Anteile an Rapamycin und nichtionischem Tensid vorherbestimmt werden, im in der Zusammensetzung die gewünschten Konzentrationen zu ergeben.

2. Verfahren, wie in Anspruch 1 beansprucht, worin das organische Lösungsmittel Aceton, Methanol oder Äthanol ist.

3. Verfahren, wie in Anspruch 1 oder Anspruch 2 beansprucht, worin das nichtionische Tensid polyoxyäthyliertes Rizinusöl oder polyoxyäthyliertes hydriertes Rizinusöl ist.

4. Verfahren, wie in Anspruch 1 oder Anspruch 2 beansprucht, worin die wässerige Lösung 1 bis 20%-Masse nichtionisches Tensid aufweist.

5. Verfahren, wie in Anspruch 1 beansprucht, worin die wässerige Lösung 1 bis 20%-Masse eines nichtionischen Tensids ausgewählt aus polyoxyäthyliertem Rizinusöl und polyoxyäthylierten hydrierten Rizinusöl ist.

**Revendications pour les Etats Contractants BE CH DE FR GB IT LI LU NL SE;**

1. Composition injectable de rapamycine, propre à l'administration intraveineuse, que comprend 1 à 20 mg de rapamycine par millilitre de composition et un surfactant non ionique choisi entre des acides gras polyoxyéthylés, des alcools gras polyoxyéthylés et des esters d'hydroxyacides gras de glycérol polyoxyéthylé.

2. Composition suivant la revendication 1, contenant une solution aqueuse de 1 à 50% en poids du surfactant non ionique.

3. Composition suivant la revendication 1, contenant une solution aqueuse de 1 à 20% en poids du surfactant non ionique.

4. Composition suivant la revendication 1 ou la revendication 2, dans laquelle le surfactant non ionique est l'huile de ricin polyoxyéthylée ou l'huile de ricin hydrogénée polyoxyéthylée.

5. Composition suivant la revendication 1, contenant 1 à 20 mg de rapamycine par millilitre d'une solution aqueuse de 1 à 20% en poids d'un surfactant non ionique choisi entre l'huile de ricin polyoxyéthylée et l'huile de ricin hydrogénée polyoxyéthylée.

6. Procédé de préparation d'une composition injectable suivant la revendication 1, qui consiste à dissoudre de la rapamycine dans un solvant organique qui est miscible au surfactant non ionique, et ou bien

(i) à ajouter le surfactant non ionique, le cas échéant en enlevant un peu ou la totalité du solvant organique et en ajoutant de l'eau; ou bien

(ii) à ajouter une solution de surfactant non ionique dans l'eau;

les quantités de rapamycine et de surfactant non ionique étant prédéterminées de manière que la composition renferme les concentrations désirées.

7. Procédé suivant la revendication 6 dans lequel le solvant organique est l'acétone, le méthanol ou l'éthanol.

8. Procédé suivant la revendication 6 ou la revendication 7 dans lequel le surfactant non ionique est l'huile de ricin polyoxyéthylée ou l'huile de ricin hydrogénée polyoxyéthylée.

9. Procédé suivant l'une quelconque des revendications 6, 7 et 8 dans lequel la solution aqueuse contient 1 à 20% en poids de surfactant non ionique.

10. Composition injectable par voie intraveineuse suivant l'une quelconque des revendications 1 à

**0 041 795**

5, destinée à être utilisée comme agent pour accroître les taux sanguins de rapamycine ou pour faire arriver la rapamycine au cerveau d'un mammifère.

**Revendications Pour l'Etat Contractant AT:**

1. Procédé de préparation d'une composition injectable, propre à l'administration intraveineuse, comprenant 1 à 20 mg de rapamycine par millilitre d'une solution aqueuse d'un surfactant non ionique choisi entre des acides gras polyoxyéthylés, des alcools gras polyoxyéthylés et des esters d'hydroxy-acides gras de glycérol polyoxyéthylés, qui consiste à dissoudre de la rapamycine dans un solvant organique qui est miscible au surfactant non ionique, et ou bien

(i) à ajouter le surfactant non ionique, le cas échéant en enlevant une partie ou la totalité du solvant organique et en ajoutant de l'eau; ou bien
(ii) à ajouter une solution de surfactant non ionique dans l'eau;

les quantités de rapamycine et de surfactant non ionique étant prédéterminées de manière que la composition renferme les concentrations désirées.

2. Procédé suivant la revendication 1, dans lequel le solvant organique est l'acétone, le méthanol ou l'éthanol.

3. Procédé suivant la revendication 1 ou la revendication 2, dans lequel le surfactant non ionique est l'huile de ricin polyoxyéthylée ou l'huile de ricin hydrogénée polyoxyéthylée.

4. Procédé suivant la revendication 1 ou la revendication 2, dans lequel la solution aqueuse comprend 1 à 20% en poids de surfactant non ionique.

5. Procédé suivant la revendication 1, dans lequel la solution aqueuse comprend 1 à 20% en poids d'un surfactant non ionique choisi entre l'huile de ricin polyoxyéthylée et l'huile de ricin hydrogénée polyoxyéthylée.

14